# EUROPEAN PATENT SPECIFICATION

(11) **EP 2 830 502 B1**
(45) Date of publication and mention of the grant of the patent: **13.07.2016**
(21) Application number: 13724381.2
(22) Date of filing: 29.03.2013
(51) Int. Cl.: A61B 6/00, G06T 5/00

(54) **ARTIFACT REMOVAL USING SHAPE SENSING**
ELIMINIERUNG VON ARTEFAKTEN MITTELS FORMMESSUNG
SUPPRESSION D'ARTÉFACT AU MOYEN DE LA DÉTECTION DE FORME

(30) Priority: 23.04.2012 US 201261617313 P
(43) Date of publication of application: 04.02.2015
(73) Proprietor: Koninklijke Philips N.V., 5656 AE Eindhoven (NL); Philips GmbH, 20099 Hamburg (DE)
(72) Inventor: GRASS, Michael, 5656 AE Eindhoven (NL); SCHÄFER, Dirk, 5656 AE Eindhoven (NL); MANZKE, Robert, 5656 AE Eindhoven (NL); CHAN, Raymond, 5656 AE Eindhoven (NL)
(74) Representative: Steffen, Thomas
(86) International application number: PCT/IB2013/052536
(87) International publication number: WO 2013/144912

(56) References cited:
- US-A1- 2010 030 063
- ZHANG XIAOMENG ET AL: "Metal artifact reduction in x-ray computed tomography (CT) by constrained optimization", MEDICAL PHYSICS, AIP, MELVILLE, NY, US, vol. 38, no. 2, 11 January 2011 (2011-01-11), pages 701-711, XP012145073, ISSN: 0094-2405, DOI: 10.1118/1.3533711 ISBN: 978-1-930524-56-9
- MATTHIAS SCHNEIDER ET AL: "Automatic global vessel segmentation and catheter removal using local geometry information and vector field integration", BIOMEDICAL IMAGING: FROM NANO TO MACRO, 2010 IEEE INTERNATIONAL SYMPOSIUM ON, IEEE, PISCATAWAY, NJ, USA, 14 April 2010 (2010-04-14), pages 45-48, XP032134258, DOI: 10.1109/ISBI.2010.5490419 ISBN: 978-1-4244-4125-9
- YONG-LAE PARK ET AL: "Real-Time Estimation of 3-D Needle Shape and Deflection for MRI-Guided Interventions", IEEE / ASME TRANSACTIONS ON MECHATRONICS, IEEE SERVICE CENTER, PISCATAWAY, NJ, US, vol. 15, no. 6, 13 December 2010 (2010-12-13), pages 906-915, XP011339830, ISSN: 1083-4435, DOI: 10.1109/TMECH.2010.2080360

## Description

This disclosure relates to medical instruments and imaging and more particularly to employing instrument shape sensing to enhance images by removing instrument artifacts from the images.

In angiographic studies during interventional procedures, catheters are used to inject contrast agent into the vascular structure of interest. Examples of such procedures include coronary angiography procedures, e.g., Percutaneous Transluminal Coronary Intervention (PTCI) or valvular procedures such as, Transcatheter Aortic-Valve Implantation (TAVI). Balloons, stents, and other filter device procedures also have catheter-based deployment and very often, there is a need for rotational X-ray (RX) imaging to acquire projections for 3D volumetric reconstruction (e.g., 3D atriography) and visualization. The contrast agent enhanced projections can be used as input for those 3D image reconstructions. While the catheter is a prerequisite to apply the contrast agent, it is not necessarily favorable to see the catheter in a reconstructed field of view. This is particularly unfavorable when the catheter is only partially inside the field of view (not visible in all projections), when the catheter is only filled with contrast agent in a part of the rotational sequence, or when the catheter shows strong motion during the acquisition.

U.S. Patent Application No. 2010/0030063 describes a system (44) with an optical tracking device coupled to an instrument. A shape sensor is coupled to the instrument for determining a shape of the instrument. A navigation system determines a position and the shape of the instrument relative to an anatomical structure based on the position of the tracking device. A display displays an image of the anatomical structure and of the shape of the instrument.

The invention is defined in the independent claims, further embodiments of the invention are defined in the dependent claims.

In accordance with the present principles, a medical system for imaging includes a processor and memory coupled to the processor. The memory includes a shape sensing module configured to receive shape sensing data from a shape sensing system coupled to a medical instrument. The shape sensing system is configured to measure a shape and position of the medical instrument. An image generation module is configured to detect and digitally remove image artifacts of the medical instrument from a generated image based on the shape and the position of the medical instrument as measured by the shape sensing system.

Another medical system includes a medical instrument and a shape sensing system coupled to the medical instrument to measure a shape and position of the medical instrument. An imaging system is configured to image a subject wherein image artifacts of the medical instrument are at least partially present in the image of the subject. An image generation module is configured to detect and digitally remove at least the artifacts of the medical instrument from a generated image based on at least the shape and the position of the medical instrument as measured by the shape sensing system.

A method for image processing includes gathering shape sensing data from an instrument; imaging the instrument in an internal image volume; detecting imaging artifacts caused by the instrument in the image volume by employing the shape sensing data from the instrument; and removing the imaging artifacts by employing an image interpolation process and the shape sensing data.

These and other objects, features and advantages of the present disclosure will become apparent from the following detailed description of illustrative embodiments thereof, which is to be read in connection with the accompanying drawings.

This disclosure will present in detail the following description of preferred embodiments with reference to the following figures wherein:
FIG. 1 is a block/flow diagram showing an image artifact removal system which employs shape sensing data to identify and remove the image artifacts in accordance with one embodiment;
FIG. 2A is a fluoroscopy image showing a catheter passing into a heart in accordance with one example;
FIG. 2B is a fluoroscopy image showing the catheter detected and highlighted by image processing in accordance with the example;
FIG. 3A is a fluoroscopy image showing catheter projection artifacts in the heart in accordance with the example;
FIG. 3B is a fluoroscopy image showing the catheter projection artifacts removed in accordance with the present principles; and
FIG. 4 is a flow diagram showing a method for image processing in accordance with an illustrative embodiment.

In accordance with the present principles, safe and real time instrument identification and removal from an image are provided. This instrument identification and removal from the image is a valuable feature as it minimizes the impact of associated imaging artifacts. Such a solution is applicable to catheters, implanted electrode leads, guidewires, etc. in an imaging field-of-view for X-rays or other radiation.

In particularly useful embodiments, optical shape sensing (OSS) is employed to detect a position, shape and orientation of an instrument. OSS utilizes special optical fibers which can be integrated into a catheter, guidewire, electrode lead, or other flexible elongated instrument and are connected to an analysis unit outside a body of a patient. The position and the shape of the fiber is measured in real time using modeling and analysis of optical Rayleigh scattering with respect to a reference in the analysis unit connected to one end of the instrument. The position of the instrument along its extent is thereby known in the imaging space. The spatial information on the shape and the position of the instrument during contrast injection and rotational projection acquisition can be used to compute the position of the instrument on the projection images and remove the instrument from the projections using an interpolation method and known instrument geometry and characteristics in imaging.

Shape sensing information, combined with an expert database or library of device characteristics (e.g., based on 3D models or on prior cases or datasets (historic data)) will permit simplified removal of the instrument's footprint or X-ray shadow within an overall projection dataset. The processing overhead related to instrument detection and tracking can be eliminated. The expert database or library can also be augmented by standard machine learning methods for adaptive optimization for the procedures at hand.

It also should be understood that the present invention will be described in terms of medical instruments; however, the teachings of the present invention are much broader and are applicable to any imaging system which may employ shape sensing to remove an instrument or object from an image. In some embodiments, the present principles are employed in tracking or analyzing complex biological or mechanical systems. In particular, the present principles are applicable to internal tracking and imaging procedures of biological systems, procedures in all areas of the body such as the lungs, gastro-intestinal tract, excretory organs, brain, heart, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD.

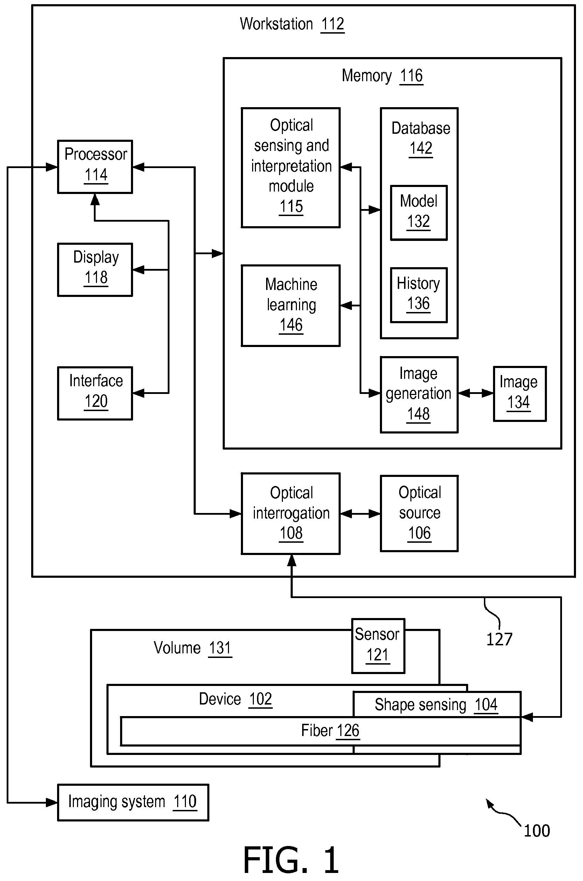

Referring now to the drawings in which like numerals represent the same or similar elements and initially to FIG. 1, a system 100 for corrective imaging is illustratively shown in accordance with one embodiment. System 100 may be employed to render images for surgical procedures where a benefit is gained by removing artifacts of an instrument or other object from the images. The system 100 may be employed for real time imaging or stored imaging for various applications, e.g., multi-planar reconstruction, etc. System 100 may include a workstation or console 112 from which a procedure is supervised and/or managed. Workstation 112 preferably includes one or more processors 114 and memory 116 for storing programs and applications. Memory 116 may store an optical sensing and interpretation module (or analysis module) 115 configured to interpret optical feedback signals from a shape sensing device or system 104. Optical sensing module 115 is configured to use the optical signal feedback (and any other feedback, e.g., electromagnetic (EM) tracking) to reconstruct deformations, deflections and other changes associated with a medical device or instrument 102 and/or its surrounding region. The medical device 102 may include a catheter, a guidewire, a probe, an endoscope, a robot, an electrode, a filter device, a balloon device, electrode lead, or other instrument or medical component, etc.

Workstation 112 may include a display 118 for viewing internal images of a subject provided by an imaging system 110. The imaging system 110 may include, e.g., a magnetic resonance imaging (MRI) system, a fluoroscopy system, a computed tomography (CT) system, ultrasound (US), etc. Display 118 may also permit a user to interact with the workstation 112 and its components and functions. This is further facilitated by an interface 120 which may include a keyboard, mouse, a joystick or any other peripheral or control to permit user interaction with the workstation 112.

The shape sensing system 104 on device 102 includes one or more optical fibers 126 which are coupled to the device 102 in a set pattern or patterns. The optical fibers 126 connect to the workstation 112 through cabling 127. The cabling 127 may include fiber optics, electrical connections, other instrumentation, etc., as needed.

Workstation 112 may include an optical source 106 to provide optical fibers 126 with light when shape sensing 104 includes optical fiber shape sensing. An optical interrogation unit 108 may also be employed to detect light returning from all fibers. This permits the determination of strains or other parameters, which will be used to interpret the shape, orientation, etc. of the interventional device 102. The light signals will be employed as feedback to make adjustments, to access errors, to determine a shape and position of the device 102 and to calibrate the device 102 (or system 100).

Shape sensing device 104 preferably includes one or more fibers 126, which are configured to exploit their geometry for detection and correction/calibration of a shape of the device 102. Shape sensing system 104 with fiber optics may be based on fiber optic Bragg grating sensors. A fiber optic Bragg grating (FBG) is a short segment of optical fiber that reflects particular wavelengths of light and transmits all others. This is achieved by adding a periodic variation of the refractive index in the fiber core, which generates a wavelength-specific dielectric mirror. A fiber Bragg grating can therefore be used as an inline optical filter to block certain wavelengths, or as a wavelength-specific reflector.

A fundamental principle behind the operation of a fiber Bragg grating is Fresnel reflection at each of the interfaces where the refractive index is changing. For some wavelengths, the reflected light of the various periods is in phase so that constructive interference exists for reflection and, consequently, destructive interference for transmission. The Bragg wavelength is sensitive to strain as well as to temperature. This means that Bragg gratings can be used as sensing elements in fiber optical sensors. In an FBG sensor, the measurand (e.g., strain) causes a shift in the Bragg wavelength.

One advantage of this technique is that various sensor elements can be distributed over the length of a fiber. Incorporating three or more cores with various sensors (gauges) along the length of a fiber that is embedded in a structure permits a three dimensional form of such a structure to be precisely determined, typically with better than 1 mm accuracy. Along the length of the fiber, at various positions, a multitude of FBG sensors can be located (e.g., 3 or more fiber sensing cores). From the strain measurement of each FBG, the curvature of the structure can be inferred at that position. From the multitude of measured positions, the total three-dimensional form is determined.

As an alternative to fiber-optic Bragg gratings, the inherent backscatter in conventional optical fiber can be exploited. One such approach is to use Rayleigh scatter in standard single-mode communications fiber. Rayleigh scatter occurs as a result of random fluctuations of the index of refraction in the fiber core. These random fluctuations can be modeled as a Bragg grating with a random variation of amplitude and phase along the grating length. By using this effect in three or more cores running within a single length of multicore fiber, the 3D shape and dynamics of the surface of interest can be followed. It should be understood that other shape sensing techniques, not limited by those described, may also be employed.

The optical fibers 126 can be integrated into the device 102 (e.g., catheter, guidewire, electrode lead, or other flexible elongated instrument) and connected to the analysis unit 115 outside a body or imaging volume 131 of a patient or subject. The position and the shape of the fiber 126 is measured in real time using modeling and analysis of the optical scattering or back reflection with respect to a reference in the analysis module 115 stored in memory 116. A position of the device 102 along its extent is thereby known in the imaging space of an imaging system 110, e.g., an X-ray system, CT system, etc.

In one embodiment, the imaging system 110 may include a rotational X-ray system, and the device 102 may include a contrast dispensing catheter. The spatial information on the shape and the position of the device 102 during contrast injection and rotational projection acquisition can be used to calculate the position of the catheter 102 in an image 134 (which may be viewed on a display 118). Since the optical sensing module 115 can accurately compute the shape and position of the device 102, an image generator 148 can use this information and other information to pinpoint image artifacts for removal from the image 134. The image generator 148 can identify and remove the device 102 (e.g., catheter) based on the shape sensing information and may employ the shape sensing information along with other information to remove image artifacts. The image generator 148 may employ a suitable interpolation method, such as image inpainting or other image processing technique to alter the pixels of the image to remove the device 102 and/or image artifacts due to the device 102 from the image 134. Knowing catheter / device geometry and characteristics of X-ray imaging, an accurate determination of the image portion which the catheter (or other device) 102 occupies can be accurately determined.

The shape sensing information, combined with an expert database or library 142 of device characteristics may be employed to more confidently identify the device 102 and its artifacts in the image 134. The database or library 142 may include 3D model(s) 132 or stored images of prior cases/ historic data 136. Models 132 may be generated based upon images taken before the device 102 has been introduced so that a comparison can be made with images having artifacts or the device 102 present. The historic data 136 may include previously collected image frames so that portions of the device 102 and their earlier trajectories can be determined and employed to predict places where artifacts may occur. The library 142 can also be augmented using a machine learning module 146 or other known learning method for adaptive optimization of images and image comparisons. The optimized images may be employed to more reliably remove artifacts of the shaped sensing tracked device 102 from the image 134 based on a current procedure, a particular patient, a particular circumstance, etc.

For example, design and development of the model 132 and/or historic data 136 may include evolving or recording behavior based on empirical or historic information such as from image data or artifact data. The machine learning module 146 can take advantage of examples (data) to capture characteristics of interest over time. Data can be seen as examples that illustrate relations between observed variables including device shapes and positions. The machine learning module 146 can automatically learn to recognize complex patterns and make intelligent decisions based on data of where instrument projections, instrument images, instrument artifacts, etc. are likely to be in the image 134. The shape sensing data makes the learning much simpler and much more reliable.

Such adaptive optimization will permit for a more simplified removal of the device footprint, artifacts, X-ray shadows, etc. within an overall projection dataset (or image 134). By employing the present principles, processing overhead associated with catheter or device detection and tracking can be eliminated.

In another embodiment, for all rotational angiographic acquisitions of moving structures, e.g., in the heart, the motion information of the catheter or device 102 tracked by the optical shape sensing system 104 can be employed to derive a physiological signal. The physiological signal may include a signal measured by a sensor device 121, e.g., corresponding to an electro-cardiogram (ECG) signal, a signal indicating the displacement of the heart due to breathing motion, or any other signal representing physical dynamic motion. This motion information can be used for gated and/or motion compensated reconstruction. The shape measurements of the shape sensing system 104 of the device 102 are correlated with the projection acquisition in space and time and motion information due to known sources can be accounted for in the image processing in the image generation module 148. Motion or lack thereof (breath hold commands, hyper pacing, and adenosine) may be accounted for or other sensor signals may be employed to collect motion data (e.g., breathing belt, ECG signal).

In an interventional setup, a shape sensing enabled device (102, 104) may be registered to X-ray (CT) imaging space. The registration may be performed using, for example, known phantom-based calibration steps. When a rotational X-ray acquisition is performed for volumetric X-ray imaging, and the shape sensing enabled device is in the X-ray field of view, for each acquired position, there is a spatial correspondence of the device visible in X-ray and its shape from shape sensing. The shape in the X-ray image may however be truncated. Due to system lag of any of the systems, there can however be a temporal discrepancy between the shape (from shape sensing) and what is visible in the X-ray. Temporal correspondence is preferable between shape sensing and the imaging. This can be achieved by time-stamping of both data and calibration of system lags or other methods such as employing a physiological signal to provide a temporal reference.

The shape sensing data and X-ray projections can be synchronized via other external signals (e.g., from extrinsic events in an interventional suite or from physiological streams such as, ECG, hemodynamic, respiratory information, etc.) or via internal clocks which allow for time annotation of continuously acquired multi-modal (shape sensing/X-ray) measurements or for triggered acquisition of these datasets in prospective or retrospective fashion. These synchronized streams permit interleaving of shape sensing data and X-ray measurements and increased accuracy of shape sensing based instrument removal from X-ray projections and subsequent 3D volumetric reconstructions.

In another embodiment, correlation between the X-ray image and the shape sensing data may be performed using scintillating fiber claddings (e.g., fiber cladding visible in fluoroscopic images) to be attached to or integrated in the optical shape sensing fiber (126). When the cladding is inside the X-ray field of view, the cladding image may be employed to temporally correlate the shape signal to the X-ray image. The shape and X-ray information do not have to run on the same clock. Intra-frame motion could even be compensated for using this method.

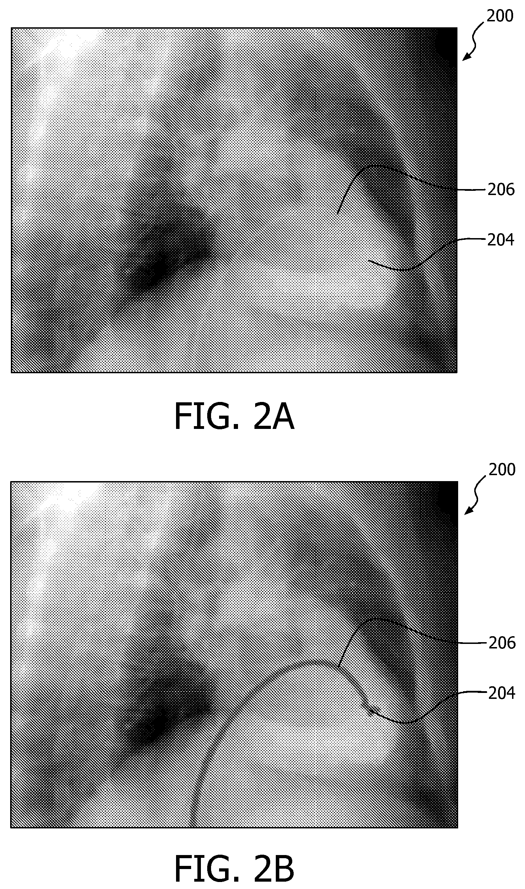

Referring to FIGS. 2A and 2B, a fluoroscopy image 200 illustratively shows an exemplary ventricular projection. It should be understood that while fluoroscopy is described here as an example, other imaging modalities may be employed instead of fluoroscopy or in addition to fluoroscopy (e.g., used in combination with other imaging modalities e.g., computed tomography (CT), magnetic resonance (MR), etc.).

FIG. 2A shows the image 200 with a catheter 202 and injection point 204 in a left ventricle. FIG. 2B shows the same projection with a detected catheter 206. The detected catheter 206 has its position and shape determined using optical shape sensing. The detected catheter 206 is highlighted by image processing techniques to improve its visibility.

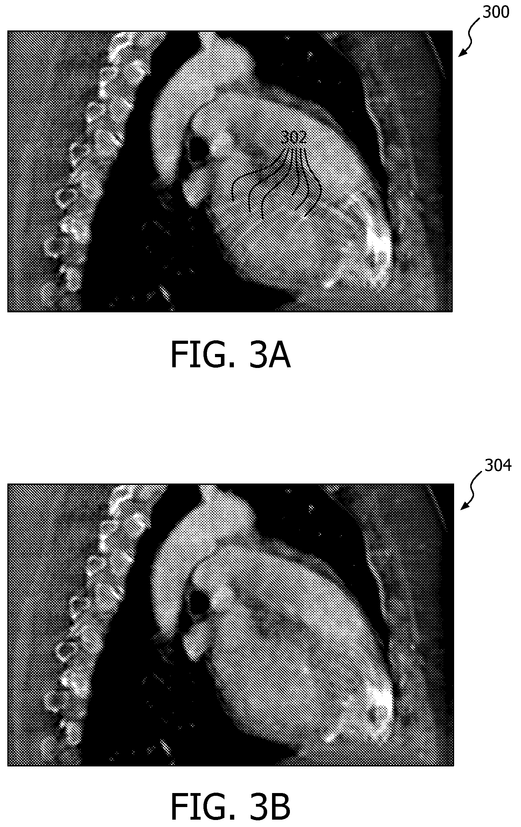

Referring to FIGS. 3A and 3B, a three dimensional rotational X-ray (3D-RX) cardiac data set image 300 is illustrative shown. In FIG. 3A, an image 300 shows a plurality of catheter projections 302 which have not been detected and removed. The projections occur as a result of rotational X-ray imaging. In FIG. 3B, an exemplary 3D-RX cardiac data set 304 is depicted after the catheter projections have been detected and removed in accordance with the present principles.

In atrial fibrillation (AFIB) or other structural heart disease procedures which employ a rotational angiography to generate 3D information, artifacts due to the bright contrast emitting catheter tip are avoided by erasing the catheter from a sequence of rotational projections 302 as illustrated in FIGS. 3A and 3B. Artifacts in the resulting tomographic images are thereby avoided. Time synchronization of the catheter tracking (using shape sensing, for example) with each X-ray projection addresses the dynamic nature of the problem. The methods can be extended to include subsequently adding of a dynamic catheter structure to a reconstructed image without introducing artifacts. This can be achieved, for example, by first removing the device and the device projections from the image (to reduce artifacts) then the device structure can be added to the reconstructed image. This is of particularly importance for implants, whose relative location to the adjacent anatomy is of interest. It should also be noted that the images may be displayed in real time so that the projections are removed and a simulation (or the actual device) can be visualized during a procedure.

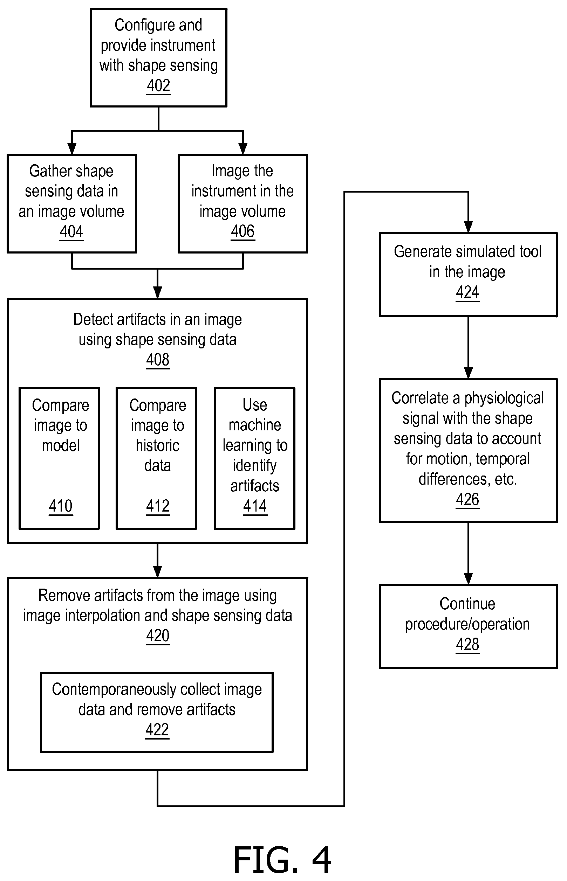

Referring to FIG. 4, a block diagram is shown to describe a method for image processing in accordance with one illustrative embodiment. In block 402, an instrument is configured to include a shape sensing system. The instrument is moved into an imaging volume where it is employed to assist in imaging the volume and is employed to perform a utilitarian function. In one illustrative embodiment, the instrument includes a catheter for injecting contrast in a heart chamber. The imaging system may include an X-ray system (e.g., CT) and in particular a rotational X-ray system. In block 404, shape sensing data is gathered from the instrument. This may be in the form of a reflected light signal that reveals the shape and position of the instrument in the imaging space. In block 406, the instrument is imaged along with an internal image volume.

During imaging, especially where radiation is employed, reflections, shadows and other artifacts may occur. In block 408, imaging artifacts caused by the instrument in the image volume are detected by employing the shape sensing data from the instrument. The shape sensing data reveals the position and the shape of the instrument in various image projections and angles. These images are reviewed in light of one or more of models, historic data, etc. and the shape sensing data to identify the artifacts.

In block 410, a model stored in memory may be compared with the image with artifacts and medical instrument geometry may also be considered in the comparison for detecting and removing of the artifacts in accordance with information from the shape sensing system. In block 412, historic data of previous events may be compared with the image with artifacts (e.g., the progression of motion of the instrument) for detection and removal of the artifacts in accordance with information from the shape sensing system. In block 414, removal of the image artifacts may be optimized by employing machine learning to better and more accurately identify the artifacts based upon earlier cases, models, etc.

In block 420, the imaging artifacts are removed by employing an image interpolation process and the shape sensing data. The image interpolation process may include an inpainting image process or other image process that can adjust pixels to remove artifacts and instrument images from a medical image or the like. In block 422, the image artifacts are preferably contemporaneously removed while collecting image data. This results in real time or near real time image correction (removal of artifacts, etc.).

In block 424, if the instrument is removed from the image (or even if it is not), a simulated image of the medical instrument may be generated for visualization in a generated image. A virtual image of the instrument, free from artifacts and based on shape sensing data, will provide a user with useful information of the shape and position of the instrument in real time or near real time. In block 426, a physiological signal may be correlated with the shape sensing data to account for patient motion. The physiological signal may include an ECG signal, which can be employed to account for heart beats. In other embodiments, a breathing sensor may account for motion due to breathing, a motion sensor may account for muscle movement, etc. In block 428, a procedure or operation continues as needed.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for artifact removal using shape sensing (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. A medical system for imaging, comprising:
a processor (114);
a shape sensing system (104) coupled to a medical instrument (102), the shape sensing system configured to measure a shape and position of the medical instrument;
an imaging system (110) configured to generate an image of a subject ; and
memory (116) coupled to the processor, the memory including:
an optical shape sensing module (115) configured to receive shape sensing data from the shape sensing system (104); **characterized in that** the system further comprises
an image generation module (148) configured to detect and digitally remove image artifacts of the medical instrument from the generated image based on at least the shape and the position of the medical instrument as measured by the shape sensing system.

2. The system as recited in claim 1, further comprising a model (132) stored in the memory and employed to compare against an image with the artifacts for detection and removal of the artifacts in accordance with information from the shape sensing system.

3. The system as recited in claim 1, further comprising historic data storage (136) of previous events stored in the memory and employed to compare against an image with the artifacts for detection and removal of the artifacts in accordance with information from the shape sensing system.

4. The system as recited in claim 1, further comprising a machine learning module (146) stored in the memory and configured to optimize identification and removal of the image artifacts.

5. The system as recited in claim 1, wherein the image generation module (148) digitally removes the image artifacts in accordance with information from the shape sensing system using an image interpolation process.

6. The system as recited in claim 5, wherein the image interpolation process includes an inpainting image process.

7. The system as recited in claim 1, wherein the image generation module (148) digitally removes the image artifacts and generates a simulated image of the medical instrument for visualization in the generated image.

8. The system as recited in claim 1, wherein the image generation module (148) digitally removes the image artifacts contemporaneously with collecting image data resulting in real time image correction.

9. The system as recited in claim 1, further comprising a sensing device (121) configured to measure a physiological signal, the physiological signal being correlated to shape sensing data to account for temporal changes.

10. A system as recited in claim 1, further comprising the medical instrument (102).

11. A method for image processing, comprising:
gathering (404) shape sensing data from an instrument; and
imaging (406) the instrument in an internal image volume; **characterized in that** the method further comprises:
detecting (408) imaging artifacts caused by the instrument in the image volume by employing the shape sensing data from the instrument; and
removing (420) the imaging artifacts by employing an image interpolation process and the shape sensing data.

12. The method as recited in claim 11, further comprising comparing (410) a model stored in memory with the image having the artifacts to detect and remove the artifacts in accordance with information from the shape sensing system.

13. The method as recited in claim 11, further comprising comparing (412) historic data of previous events with the image having the artifacts to detect and remove the artifacts in accordance with information from the shape sensing system.

14. The method as recited in claim 11, wherein the image artifacts are contemporaneously removed with collecting image data, resulting in real time image correction.

15. The method as recited in claim 11, further comprising correlating (426) a physiological signal with the shape sensing data to account for temporal changes.

## Patentansprüche

1. Medizinisches System zur Bildgebung, das Folgendes umfasst:
einen Prozessor (114);
ein Formmesssystem (104), das mit einem medizinischen Instrument (102) gekoppelt ist, wobei das Formmesssystem konfiguriert ist, um eine Form und Position des medizinischen Instruments zu messen;
ein Bildgebungssystem (110), das konfiguriert ist, um ein Bild eines Patienten zu erzeugen; und
Speicher (116), der mit dem Prozessor gekoppelt ist, wobei der Speicher Folgendes umfasst:
ein optisches Formmessmodul (115), das konfiguriert ist, um die Formmessdaten von dem Formmesssystem (104) zu empfangen; **dadurch gekennzeichnet, dass** das System weiterhin Folgendes umfasst:
ein Bilderzeugungsmodul (148), das konfiguriert ist, um Bildartefakte des medizinischen Instruments basierend auf mindestens der Form und der Position des medizinischen Instruments, wie sie durch das Formmesssystem gemessen wurden, zu detektieren und digital aus dem erzeugten Bild zu entfernen.

2. System nach Anspruch 1, weiterhin umfassend ein Modell (132), das in dem Speicher gespeichert ist und genutzt wird, um mit einem artefaktbehafteten Bild verglichen zu werden, um die Artefakte in Übereinstimmung mit Informationen aus dem Formmesssystem zu detektieren und zu entfernen.

3. System nach Anspruch 1, weiterhin umfassend einen Speicher (136) für historische Daten früherer Ereignisse, die in dem Speicher gespeichert sind und genutzt werden, um mit einem artefaktbehafteten Bild verglichen zu werden, um die Artefakte in Übereinstimmung mit Informationen aus dem Formmesssystem zu detektieren und zu entfernen.

4. System nach Anspruch 1, weiterhin umfassend ein Maschinenlernmodul (146), das in dem Speicher gespeichert ist und konfiguriert ist, um das Identifizieren und Entfernen von Bildartefakten zu optimieren.

5. System nach Anspruch 1, wobei das Bilderzeugungsmodul (148) die Bildartefakte in Übereinstimmung mit Informationen aus dem Formmesssystem unter Verwendung eines Bildinterpolationsprozesses digital entfernt.

6. System nach Anspruch 5, wobei der Bildinterpolationsprozess einen Vermalungs-Bildprozess umfasst.

7. System nach Anspruch 1, wobei das Bilderzeugungsmodul (148) die Bildartefakte digital entfernt und ein simuliertes Bild des medizinischen Instruments zur Visualisierung in dem erzeugten Bild erzeugt.

8. System nach Anspruch 1, wobei das Bilderzeugungsmodul (148) die Bildartefakte zeitgleich mit dem Erfassen von Bilddaten digital entfernt, wodurch sich eine Echtzeit-Bildkorrektur ergibt.

9. System nach Anspruch 1, weiterhin umfassend eine Messvorrichtung (121), die konfiguriert ist, um ein physiologisches Signal zu messen, wobei das physiologische Signal mit den Formmessdaten korreliert ist, um zeitliche Veränderungen zu berücksichtigen.

10. System nach Anspruch 1, das weiterhin das medizinische Instrument (102) umfasst.

11. Verfahren zur Bildverarbeitung, das Folgendes umfasst:
Erfassen (404) von Formmessdaten von einem Instrument; und
Darstellen (406) des Instruments in einem internen Bildvolumen; **dadurch gekennzeichnet, dass** das Verfahren weiterhin Folgendes umfasst:
Detektieren (408) von durch das Instrument verursachten Bildgebungsartefakten in dem Bildvolumen durch Nutzen der Formmessdaten von dem Instrument; und
Entfernen (420) der Bildgebungsartefakte durch Nutzen eines Bildinterpolationsprozesses und der Formmessdaten.

12. Verfahren nach Anspruch 11, weiterhin umfassend das Vergleichen (410) eines im Speicher gespeicherten Modells mit dem artefaktbehafteten Bild, um die Artefakte in Übereinstimmung mit Informationen von dem Formmesssystem zu detektieren und zu entfernen.

13. Verfahren nach Anspruch 11, weiterhin umfassend das Vergleichen (412) von historischen Daten früherer Ereignisse mit dem artefaktbehafteten Bild, um die Artefakte in Übereinstimmung mit Informationen von dem Formmesssystem zu detektieren und zu entfernen.

14. Verfahren nach Anspruch 11, wobei die Bildartefakte zeitgleich mit dem Erfassen von Bilddaten entfernt werden, wodurch sich eine Echtzeit-Bildkorrektur ergibt.

15. Verfahren nach Anspruch 11, weiterhin umfassend das Korrelieren (426) eines physiologischen Signals mit den Formmessdaten, um zeitliche Veränderungen zu berücksichtigen.

## Revendications

1. Système médical d'imagerie, comprenant :
un processeur (114) ;
un système de détection de forme (104) couplé à un instrument médical (102), le système de détection de forme étant configuré pour mesurer une forme et une position de l'instrument médical ;
un système d'imagerie (110) configuré pour générer une image d'un sujet ;
et
une mémoire (116) couplée au processeur, la mémoire incluant :
un module de détection de forme optique (115) configuré pour recevoir des données de détection de forme en provenance du système de détection de forme (104) ; **caractérisé en ce que** le système comprend en outre
un module de génération d'image (148) configuré pour détecter et supprimer numériquement des artéfacts d'image de l'instrument médical de l'image générée sur la base d'au moins la forme et la position de l'instrument médical telles que mesurées par le système de détection de forme.

2. Système selon la revendication 1, comprenant en outre un modèle (132) stocké dans la mémoire et employé pour comparer à une image avec les artéfacts en vue de la détection et de la suppression des artéfacts conformément à des informations provenant du système de détection de forme.

3. Système selon la revendication 1, comprenant en outre un stockage de données historiques (136) d'événements antérieurs stockées dans la mémoire et employées pour comparer à une image avec les artéfacts en vue de la détection et de la suppression des artéfacts conformément à des informations provenant du système de détection de forme.

4. Système selon la revendication 1, comprenant en outre un module d'apprentissage de machine (146) stocké dans la mémoire et configuré pour optimiser l'identification et la suppression des artéfacts d'image.

5. Système selon la revendication 1, dans lequel le module de génération d'image (148) supprimer numériquement les artéfacts d'image conformément à des informations provenant du système de détection de forme en utilisant un processus d'interpolation d'image.

6. Système selon la revendication 5, dans lequel le processus d'interpolation d'image inclut un processus d'image de retouche.

7. Système selon la revendication 1, dans lequel le module de génération d'image (148) supprime numériquement les artéfacts d'image et génère une image simulée de l'instrument médical en vue d'une visualisation dans l'image générée.

8. Système selon la revendication 1, dans lequel le module de génération d'image (148) supprime numériquement les artéfacts d'image en même temps que la collecte de données d'image résultant dans une correction d'image en temps réel.

9. Système selon la revendication 1, comprenant en outre un dispositif de détection (121) configuré pour mesurer un signal physiologique, le signal physiologique étant corrélé à des données de détection de forme pour représenter des changements temporaux.

10. Système selon la revendication 1, comprenant en outre l'instrument médical (102).

11. Procédé pour traitement d'image, comprenant :
le rassemblement (404) de données de détection de forme en provenance d'un instrument ; et
l'imagerie (406) de l'instrument dans un volume d'image interne ;
**caractérisé en ce que** le procédé comprend en outre :
la détection (408) d'artéfacts d'imagerie causés par l'instrument dans le volume d'image en employant les données de détection de forme provenant de l'instrument ; et
la suppression (420) des artéfacts d'imagerie en employant un processus d'interpolation d'image et les données de détection de forme.

12. Procédé selon la revendication 11, comprenant en outre la comparaison (410) d'un modèle stocké dans une mémoire avec l'image ayant les artéfacts afin de détecter et de supprimer les artéfacts conformément à des informations provenant du système de détection de forme.

13. Procédé selon la revendication 11, comprenant en outre la comparaison (412) de données historiques d'événements antérieurs avec l'image ayant les artéfacts afin de détecter et de supprimer les artéfacts conformément à des informations provenant du système de détection de forme.

14. Procédé selon la revendication 11, dans lequel les artéfacts d'image sont supprimés en même temps que la collecte de données d'image, résultant dans une correction d'image en temps réel.

15. Procédé selon la revendication 11, comprenant en outre la corrélation (426) d'un signal physiologique avec les données de détection de forme pour représenter des changements temporaux.
